# EUROPEAN PATENT SPECIFICATION

(11) **EP 3 551 768 B1**
(45) Date of publication and mention of the grant of the patent: **06.03.2024**
(21) Application number: 17822947.2
(22) Date of filing: 12.12.2017
(51) Int. Cl.: C12Q 1/6844, C12Q 1/6853, C12Q 1/6855, C12N 15/10

(54) **METHODS FOR TAGGING AND AMPLIFYING RNA TEMPLATE MOLECULES FOR PREPARING SEQUENCING LIBRARIES**
VERFAHREN ZUR MARKIERUNG UND AMPLIFIZIERUNG VON RNA-TEMPLATE-MOLEKÜLEN ZUR HERSTELLUNG VON SEQUENZIERUNGSBIBLIOTHEKEN
PROCÉDÉS DE MARQUAGE ET D'AMPLIFICATION DE MOLÉCULES MATRICES D'ARN DESTINÉS À PRÉPARER DES BIBLIOTHÈQUES DE SÉQUENÇAGE

(30) Priority: 12.12.2016 US 201662433172 P
(43) Date of publication of application: 16.10.2019
(73) Proprietor: Grail, LLC, Menlo Park, CA 94025 (US)
(72) Inventor: LARSON, Matthew, Menlo Park, CA 94025 (US)
(74) Representative: Murgitroyd & Company
(86) International application number: PCT/US2017/065811
(87) International publication number: WO 2018/111872

(56) References cited:
- EP-A1- 1 616 947
- EP-A1- 2 684 954
- WO-A2-2010/117620
- DE-A1- 19 920 611

## Description

### FIELD OF THE INVENTION

The present invention relates to methods for tagging and amplifying nucleic acid template molecules to prepare sequencing libraries.

### BACKGROUND OF THE INVENTION

Analysis of circulating cell-free nucleic acids (e.g., cell-free DNA (cfDNA) and cell-free RNA (cfRNA)) using next generation sequencing (NGS) is recognized as a valuable diagnostic tool for many diseases. WO2010/117620 discloses a method in which an oligo comprising a unique identifier and being complementary to the C-tail of a cDNA produced from a RNA is hybridised to the cDNA and the cDNA is extended by a template switch reaction to contain the identifier. The RNA/cDNA hybrid is cleaved and bound to a sequencing adaptor. Current protocols for preparing a sequencing library from a cell-free nucleic acid sample (e.g., a plasma sample) typically involve isolating a single nucleic acid populations (i.e., cfDNA or cfRNA) for preparation of a sequencing library for analysis. However, as is well known in the art, cell-free RNA tends to be present only at low levels in test samples (typically 10 ng or less). Accordingly, there is a need in the art for new methods for preparing sequencing libraries from cell-free RNA (cfRNA).

### SUMMARY OF THE INVENTION

Aspects of the invention include methods for preparing sequencing libraries comprising a plurality of RNA molecules. In one embodiment, the present invention is directed to a method for preparing a sequencing library from a test sample comprising RNA, the method comprising the steps: (a) obtaining a test sample comprising RNA sequences, and purifying the RNA sequences from the test sample; (b) synthesizing first complementary DNA (cDNA) strands based on the RNA sequences and C-tailing 3'-ends of cDNA strands; (c) annealing a complementary template switching oligonucleotide to the C-tail of the cDNA and ligating the complementary template switching oligonucleotide to the 5'-ends of the RNA sequences to produce RNA templates; and (d) synthesizing a plurality of cDNA strands from the RNA templates using a strand- displacement reverse transcriptase. One or more steps of the method may be carried out in a single reaction step. For example, steps (b) through (d) may be carried out in a single reaction tube utilizing a reaction mixture comprising RNA primers (e.g., random hexamer RNA primers, polyT primers, or a combination thereof), a strand- displacement reverse transcriptase (e.g., MMLV reverse transcriptase), an RNA ligase (e.g., T4 RNA ligase), and optionally, a polynucleotide kinase (e.g., T4 polynucleotide kinase).

Also disclosed herein, but not encompassed within the scope of claims, but nevertheless considered useful for understanding the invention, is a method for preparing a sequencing library from a test sample comprising RNA, the method comprising the steps: (a) obtaining a test sample comprising one or more RNA sequences, and purifying the one or more RNA sequences from the test sample; (b) annealing a first RNA primer to the one or more RNA sequences; (c) extending the first RNA primer in a first nucleic acid extension reaction using reverse transcriptase, wherein the reverse transcriptase comprises reverse transcription and terminal transferase activities, to generate a plurality of DNA sequences complementary to the one or more RNA templates, and wherein the complementary DNA (cDNA) sequences further comprise a plurality of non-templated bases at the 3'-end of the cDNA sequences; (d) annealing a complementary nucleic acid sequence to the non-templated bases at the 3'-end of the cDNA sequence, wherein the complementary nucleic acid sequence further comprises a unique molecular identifier (UMI) or a unique sequence tag; (e) ligating the complementary nucleic acid sequence to the 5'-end of the one or more RNA sequences to generate one or more RNA templates, wherein the one or more RNA templates comprise the original one or more RNA sequences covalently linked to the complementary nucleic acid sequence comprising the UMI or unique sequence tag; (f) annealing one or more second RNA primers to the one or more RNA template; and (g) extending the one or more second RNA primers in a second nucleic acid extension reaction using a strand-displacement reverse transcriptase to generate a plurality of DNA sequence complementary to the one or more RNA templates, wherein the plurality of complementary DNA (cDNA) sequences each comprise the complementary DNA sequence and a UMI or unique sequence tag.

One or more steps of the method may be carried out in a single reaction step. For example, in some embodiments, steps (b) through (g) may be carried out in a single reaction tube utilizing a reaction mixture comprising RNA primers (e.g., random hexamer RNA primers, polyT primers, or a combination thereof), a strand- displacement reverse transcriptase (e.g., MMLV reverse transcriptase), an RNA ligase (e.g., T4 RNA ligase), and optionally, a polynucleotide kinase (e.g., T4 polynucleotide kinase).

Also disclosed herein, but not explicitly claimed, is a method that involves preparing a sequencing library from a test sample comprising RNA molecules, the method comprising the steps: (a) obtaining a test sample comprising one or more RNA sequences, and purifying the one or more RNA sequences from the test sample; (b) annealing a first RNA primer to the one or more RNA sequences; (c) extending the first RNA primer in a first nucleic acid extension reaction using a reverse transcriptase, wherein the reverse transcriptase comprises reverse transcription and terminal transferase activities, to generate a plurality of DNA sequences complementary to the one or more RNA sequences, wherein the terminal transferase activity adds a cytosine (C) tail to the 3'-end of the complementary DNA (cDNA) sequences; (d) annealing a template switching oligonucleotide to the 3'-cytosine tail of the cDNA sequence, wherein the template switching oligonucleotide comprises a unique molecular identifier (UMI) or a unique sequence tag; (e) ligating the template switching oligonucleotide to the 5'-end of the one or more RNA sequences with T4 RNA ligase to generate one or more RNA templates, wherein the one or more RNA templates comprise the original one or more RNA sequences covalently linked to the template switching oligonucleotide and the UMI or unique sequence tag; (f) annealing a plurality of second RNA primers to the one or more RNA templates; and (g) extending the plurality of second RNA primers in a second nucleic acid extension reaction using a strand-displacement reverse transcriptase to generate a plurality of DNA sequence complementary to the one or more RNA templates, wherein the plurality of complementary DNA (cDNA) each comprise the complementary DNA sequence and a UMI or unique sequence tag. In some embodiments, one or more steps of a method can be carried out in a single reaction step. For example, steps (b) through (g) may be carried out in a single reaction tube utilizing a reaction mixture comprising RNA primers (e.g., random hexamer RNA primers, polyT primers, or a combination thereof), a strand-displacement reverse transcriptase (e.g., MMLV reverse transcriptase), an RNA ligase (e.g., T4 RNA ligase), and optionally, a polynucleotide kinase (e.g., T4 polynucleotide kinase).

### BRIEF DESCRIPTION OF THE DRAWINGS

FIG. 1 is a flow diagram illustrating a method for tagging and amplifying RNA sequences obtained from a test sample for preparation of a sequencing library in accordance with one embodiment of the present invention;
FIG. 2 is a flow diagram illustrating a method for tagging and amplifying RNA sequences obtained from a test sample for preparation of a sequencing library; and
FIG. 3 shows pictorially the steps of a method for tagging and amplifying RNA sequences obtained from a test sample for preparation of a sequencing library.

### DEFINITIONS

It is to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit, unless the context clearly dictates otherwise, between the upper and lower limit of that range and any other stated or intervening value in that stated range is encompassed within the invention. The upper and lower limits of these smaller ranges may independently be included in the smaller ranges encompassed within the invention, subject to any specifically excluded limit in the stated range.

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), provides one skilled in the art with a general guide to many of the terms used in the present application, as do the following: Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Abbas et al, Cellular and Molecular Immunology, 6th edition (Saunders, 2007).

The term "amplicon" as used herein means the product of a polynucleotide amplification reaction; that is, a clonal population of polynucleotides, which may be single stranded or double stranded, which are replicated from one or more starting sequences. The one or more starting sequences may be one or more copies of the same sequence, or they may be a mixture of different sequences. Preferably, amplicons are formed by the amplification of a single starting sequence. Amplicons may be produced by a variety of amplification reactions whose products comprise replicates of the one or more starting, or target, nucleic acids. In one aspect, amplification reactions producing amplicons are "template-driven" in that base pairing of reactants, either nucleotides or oligonucleotides, have complements in a template polynucleotide that are required for the creation of reaction products. In one aspect, template-driven reactions are primer extensions with a nucleic acid polymerase, or oligonucleotide ligations with a nucleic acid ligase. Such reactions include, but are not limited to, polymerase chain reactions (PCRs), linear polymerase reactions, nucleic acid sequence-based amplification (NASBAs), rolling circle amplifications, and the like, disclosed in the following references: Mullis et al, U.S. Pat. Nos. 4,683,195; 4,965,188; 4,683,202; 4,800,159 (PCR); Gelfand et al, U.S. Pat. No. 5,210,015 (real-time PCR with "taqman" probes); Wittwer et al, U.S. Pat. No. 6,174,670; Kacian et al, U.S. Pat. No. 5,399,491 ("NASBA"); Lizardi, U.S. Pat. No. 5,854,033; Aono et al, Japanese patent publ. JP 4-262799 (rolling circle amplification); and the like. In one aspect, amplicons of the invention are produced by PCRs. An amplification reaction may be a "real-time" amplification if a detection chemistry is available that permits a reaction product to be measured as the amplification reaction progresses, e.g., "real-time PCR", or "real-time NASBA" as described in Leone et al, Nucleic Acids Research, 26: 2150-2155 (1998), and like references.

As used herein, the term "amplifying" means performing an amplification reaction. A "reaction mixture" means a solution containing all the necessary reactants for performing a reaction, which may include, but is not be limited to, buffering agents to maintain pH at a selected level during a reaction, salts, co-factors, scavengers, and the like.

The terms "fragment" or "segment", as used interchangeably herein, refer to a portion of a larger polynucleotide molecule. A polynucleotide, for example, can be broken up, or fragmented into, a plurality of segments, either through natural processes, as is the case with, e.g., cfDNA fragments that can naturally occur within a biological sample, or through in vitro manipulation. Various methods of fragmenting nucleic acids are well known in the art. These methods may be, for example, either chemical or physical or enzymatic in nature. Enzymatic fragmentation may include partial degradation with a DNase; partial depurination with acid; the use of restriction enzymes; intron-encoded endonucleases; DNA-based cleavage methods, such as triplex and hybrid formation methods, that rely on the specific hybridization of a nucleic acid segment to localize a cleavage agent to a specific location in the nucleic acid molecule; or other enzymes or compounds which cleave a polynucleotide at known or unknown locations. Physical fragmentation methods may involve subjecting a polynucleotide to a high shear rate. High shear rates may be produced, for example, by moving DNA through a chamber or channel with pits or spikes, or forcing a DNA sample through a restricted size flow passage, e.g., an aperture having a cross sectional dimension in the micron or submicron range. Other physical methods include sonication and nebulization. Combinations of physical and chemical fragmentation methods may likewise be employed, such as fragmentation by heat and ion-mediated hydrolysis. See, e.g., Sambrook et al., "Molecular Cloning: A Laboratory Manual," 3rd Ed. Cold Spring Harbor Laboratory Press, Cold Spring Harbor, N. Y. (2001) ("Sambrook et al.). These methods can be optimized to digest a nucleic acid into fragments of a selected size range.

The terms "polymerase chain reaction" or "PCR", as used interchangeably herein, mean a reaction for the in vitro amplification of specific DNA sequences by the simultaneous primer extension of complementary strands of DNA. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors that are well-known to those of ordinary skill in the art, e.g., exemplified by the following references: McPherson et al, editors, PCR: A Practical Approach and PCR2: A Practical Approach (IRL Press, Oxford, 1991 and 1995, respectively). For example, in a conventional PCR using Taq DNA polymerase, a double stranded target nucleic acid may be denatured at a temperature >90° C, primers annealed at a temperature in the range 50-75° C, and primers extended at a temperature in the range 72-78° C. The term "PCR" encompasses derivative forms of the reaction, including, but not limited to, RT-PCR, real-time PCR, nested PCR, quantitative PCR, multiplexed PCR, and the like. The particular format of PCR being employed is discernible by one skilled in the art from the context of an application. Reaction volumes can range from a few hundred nanoliters, e.g., 200 nL, to a few hundred µL, e.g., 200 µL. "Reverse transcription PCR," or "RT-PCR," means a PCR that is preceded by a reverse transcription reaction that converts a target RNA to a complementary single stranded DNA, which is then amplified, an example of which is described in Tecott et al, U.S. Pat. No. 5,168,038. "Real-time PCR" means a PCR for which the amount of reaction product, i.e., amplicon, is monitored as the reaction proceeds. There are many forms of real-time PCR that differ mainly in the detection chemistries used for monitoring the reaction product, e.g., Gelfand et al, U.S. Pat. No. 5,210,015 ("taqman"); Wittwer et al, U.S. Pat. Nos. 6,174,670 and 6,569,627 (intercalating dyes); Tyagi et al, U.S. Pat. No. 5,925,517 (molecular beacons). Detection chemistries for real-time PCR are reviewed in Mackay et al, Nucleic Acids Research, 30: 1292-1305 (2002). "Nested PCR" means a two-stage PCR wherein the amplicon of a first PCR becomes the sample for a second PCR using a new set of primers, at least one of which binds to an interior location of the first amplicon. As used herein, "initial primers" in reference to a nested amplification reaction mean the primers used to generate a first amplicon, and "secondary primers" mean the one or more primers used to generate a second, or nested, amplicon. "Asymmetric PCR" means a PCR wherein one of the two primers employed is in great excess concentration so that the reaction is primarily a linear amplification in which one of the two strands of a target nucleic acid is preferentially copied. The excess concentration of asymmetric PCR primers may be expressed as a concentration ratio. Typical ratios are in the range of from 10 to 100. "Multiplexed PCR" means a PCR wherein multiple target sequences (or a single target sequence and one or more reference sequences) are simultaneously carried out in the same reaction mixture, e.g., Bernard et al, Anal. Biochem., 273: 221-228 (1999)(two-color real-time PCR). Usually, distinct sets of primers are employed for each sequence being amplified. Typically, the number of target sequences in a multiplex PCR is in the range of from 2 to 50, or from 2 to 40, or from 2 to 30. "Quantitative PCR" means a PCR designed to measure the abundance of one or more specific target sequences in a sample or specimen. Quantitative PCR includes both absolute quantitation and relative quantitation of such target sequences. Quantitative measurements are made using one or more reference sequences or internal standards that may be assayed separately or together with a target sequence. The reference sequence may be endogenous or exogenous to a sample or specimen, and in the latter case, may comprise one or more competitor templates. Typical endogenous reference sequences include segments of transcripts of the following genes: β-actin, GAPDH, β₂-microglobulin, ribosomal RNA, and the like. Techniques for quantitative PCR are well-known to those of ordinary skill in the art, as exemplified in the following references: Freeman et al, Biotechniques, 26: 112-126 (1999); Becker-Andre et al, Nucleic Acids Research, 17: 9437-9447 (1989); Zimmerman et al, Biotechniques, 21: 268-279 (1996); Diviacco et al, Gene, 122: 3013-3020 (1992); and Becker-Andre et al, Nucleic Acids Research, 17: 9437-9446 (1989).

The term "primer" as used herein means an oligonucleotide, either natural or synthetic, that is capable, upon forming a duplex with a polynucleotide template, of acting as a point of initiation of nucleic acid synthesis and being extended from its 3'-end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually, primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following reference: Dieffenbach, editor, PCR Primer: A Laboratory Manual, 2nd Edition (Cold Spring Harbor Press, New York, 2003).

The terms "unique sequence tag", "sequence tag", "tag" or "barcode", as used interchangeably herein, refer to an oligonucleotide that is attached to a polynucleotide or template molecule and is used to identify and/or track the polynucleotide or template in a reaction or a series of reactions. A sequence tag may be attached to the 3'- or 5'-end of a polynucleotide or template, or it may be inserted into the interior of such polynucleotide or template to form a linear conjugate, sometimes referred to herein as a "tagged polynucleotide," or "tagged template," or the like. Sequence tags may vary widely in size and compositions; the following references provide guidance for selecting sets of sequence tags appropriate for particular embodiments: Brenner, U.S. Pat. No. 5,635,400; Brenner and Macevicz, U.S. Pat. No. 7,537,897; Brenner et al, Proc. Natl. Acad. Sci., 97: 1665-1670 (2000); Church et al, European patent publication 0 303 459; Shoemaker et al, Nature Genetics, 14: 450-456 (1996); Morris et al, European patent publication 0799897A1; Wallace, U.S. Pat. No. 5,981,179; and the like. Lengths and compositions of sequence tags can vary widely, and the selection of particular lengths and/or compositions depends on several factors including, without limitation, how tags are used to generate a readout, e.g., via a hybridization reaction or via an enzymatic reaction, such as sequencing; whether they are labeled, e.g., with a fluorescent dye or the like; the number of distinguishable oligonucleotide tags required to unambiguously identify a set of polynucleotides, and the like, and how different the tags of a particular set must be in order to ensure reliable identification, e.g., freedom from cross hybridization or misidentification from sequencing errors. In one aspect, sequence tags can each have a length within a range of from about 2 to about 36 nucleotides, or from about 4 to about 30 nucleotides, or from about 4 to about 20 nucleotides, or from about 8 to about 20 nucleotides, or from about 6 to about 10 nucleotides. In one aspect, sets of sequence tags are used, wherein each sequence tag of a set has a unique nucleotide sequence that differs from that of every other tag of the same set by at least two bases; in another aspect, sets of sequence tags are used wherein the sequence of each tag of a set differs from that of every other tag of the same set by at least three bases.

The terms "subject" and "patient" are used interchangeably herein and refer to a human or non-human animal who is known to have, or potentially has, a medical condition or disorder, such as, e.g., a cancer.

The term "sequence read" as used herein refers to nucleotide sequences read from a sample obtained from a subject. Sequence reads can be obtained through various methods known in the art.

The term "circulating tumor DNA" or "ctDNA" refers to nucleic acid fragments that originate from tumor cells or other types of cancer cells, which may be released into a subject's bloodstream as a result of biological processes, such as apoptosis or necrosis of dying cells, or may be actively released by viable tumor cells.

### DETAILED DESCRIPTION OF THE INVENTION

Aspects of the invention include methods for preparing sequencing libraries comprising a plurality of RNA molecules. In the claimed invention, the methods involve tagging and amplifying RNA template molecules in a sample for preparation of a sequencing library. In some methods, the methods utilize a reverse transcriptase enzyme with strand-displacement and terminal transferase activity, random primers (e.g., random hexamer primers, polyT primers, or a combination thereof), and a ligase reaction to create multiple cDNA copies of each RNA template molecule in a sample, wherein each cDNA molecule is tagged with a unique tagging sequence (e.g., a unique molecular index (UMI)) that is specific to the original template RNA molecule in the sample.

Methods in accordance with embodiments of the invention can be used to prepare an RNA sequencing library from a low-input (e.g., about 10 ng or less of RNA) RNA-containing test sample. For example, in some embodiments, a method can be used to prepare an RNA sequencing library from a cell-free nucleic acid (cfNA) sample containing RNA sequences. In some methods disclosed herein, a method can be used as one step in a method for preparing a sequencing library from a combined RNA and DNA cell-free nucleic acid sample.

In one embodiment, a method involves preparing a sequencing library from a test sample comprising RNA molecules or sequences, the method comprising the steps: (a) obtaining a test sample comprising RNA sequences, and purifying the RNA sequences from the test sample; (b) synthesizing first complementary DNA (cDNA) strands based on the RNA sequences and C-tailing 3'-ends of cDNA strands; (c) annealing a complementary template switching oligonucleotide to the C-tail of the cDNA and ligating the complementary template switching oligonucleotide to the 5'-ends of the RNA sequences to produce RNA templates; and (d) synthesizing a plurality of cDNA strands from the RNA templates using a strand- displacing reverse transcriptase. Some methods involve thermal cycling of the sample after an initial round of cDNA synthesis and adapter ligation to remove a first cDNA and to facilitate synthesis of a further cDNA molecule (e.g., a second, third or fourth cDNA molecule).

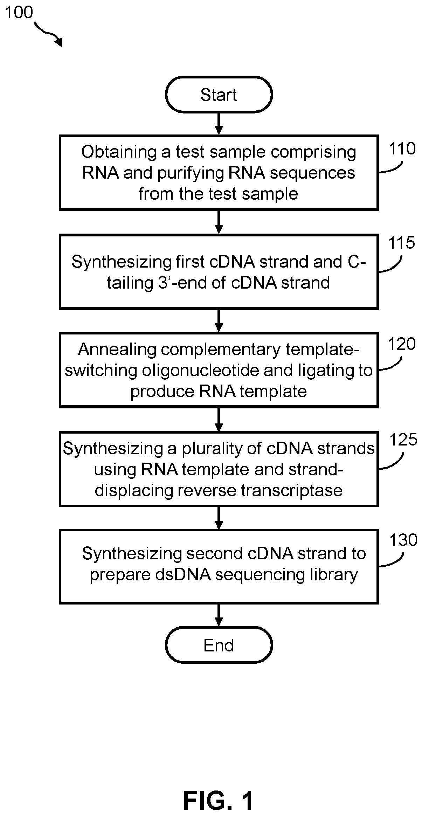

**FIG. 1** is a flow diagram illustrating an example of a method 100 for tagging and amplifying RNA sequences obtained from a test sample for preparation of a sequencing library in accordance with one embodiment of the present invention. As shown, method 100 may include, but is not limited to, the following steps.

In step 110, an RNA containing test sample is obtained and RNA sequences purified from a test sample. In general, any known method in the art can be used for purifying RNA sequences from the test sample. The subject methods can be carried out on any suitable biological sample, as described in detail herein. In some embodiments, a test sample may be a biological sample selected from the group consisting of: blood, plasma, serum, urine, fecal, and saliva samples. In one preferred embodiment, a test sample is a blood sample. In one preferred embodiment, a test sample is a plasma sample. In one embodiment, the RNA sequences comprise cell-free RNA. Optionally, the 5'-ends of the RNA sequences are phosphorylated using T4 polynucleotide kinase. In another method, the RNA sequences may be fragmented after purification of the RNA sequences from the test sample.

In step 115, a first complementary DNA (cDNA) strand is synthesized from the RNA sequences and the cDNA strands C-tailed at the 3'-ends. cDNA strands are synthesized and C-tailed using a reverse transcriptase having both reverse transcription and terminal transferase activities (e.g., C-tailing activity). In another method, the reverse transcriptase has both strand-displacement and terminal transferase (e.g., C-tailing) activities. For example, in one embodiment, MMLV reverse transcriptase (available from Clontech) is used. In accordance with the present invention, reverse transcription primers are annealed to the RNA sequences and extended by reverse transcriptase to synthesize cDNA. In one embodiment, the reverse transcription primers can be RNA primers (e.g., random hexamer primers, polyT primers, or a combination thereof). Although not wishing to be bound by theory, it is believed that the use of random hexamer primers in the reverse transcription reaction allows all RNA molecules in the sample to be captured and used as template molecules for synthesis of first strand cDNA. Random priming may also provide for greater coverage of a single RNA molecule.

In step 120, a complementary template-switching oligonucleotide is annealed (or hybridized to) the 3' C-tails of the cDNA sequences and subsequently ligated, using an RNA ligase, to the 5'-ends of the RNA sequences to produce RNA templates. The switch oligonucleotide may include, for example, a complementary hybridization sequence (e.g., a poly-G tail), a unique sequence tag (e.g., a UMI sequence), and/or a universal primer sequence for initiating second strand cDNA synthesis. In general, any known RNA ligase can be used to ligate the template-switching oligonucleotide to the 5'-ends of the RNA sequence. In one embodiment, the RNA ligase is T4 RNA ligase (available from New England BioLabs). As one of skill in the art would appreciate, the ligation of the template switch DNA oligonucleotide to the 5'-ends of the RNA molecule requires the 5' end to be phosphorylated. Accordingly, a polynucleotide kinase (e.g., a T4 polynucleotide kinase) may be utilized for phosphorylation of the 5'-end.

The complementary template-switching oligonucleotide can include a unique sequence tag (e.g., a barcode or UMI). Unique sequence tags can serve many functions. Unique sequence tags can include molecular barcode sequences, unique molecular identifier (UMI) sequences, or index sequences. Unique sequence tags (e.g., barcode or index sequences) can be used to identify individual RNA sequences originating from a common test sample such as a sample type, tissue, patient, or individual. The unique sequence tag can be a unique molecular identifier (UMI), and can be used to identify a unique RNA sequence from a test sample (e.g., from mixed cfRNA sample). The UMI sequence or tag can be used to reduce errors introduced in subsequent steps of amplification, library preparation, and sequencing. For example, the UMI can be used to reduce amplification bias, which is the asymmetric amplification of different targets due to differences in nucleic acid composition (e.g., high GC content). The unique sequence tags (UMIs) may also be used to identify, and correct for, nucleic acid mutations that arise during amplification, library preparation, or sequencing (i.e., systematic errors). The unique sequence tags (e.g., barcodes or index sequences) can be used for multiplex sequencing. Unique sequence tags can range in size from about 4 to about 20 nucleic acids in length, such as about 5, 6, 7, 8, 9, 10, 11, 12, 13, 14, 15, 16, 17, 18, or 19 nucleic acids in length.

In step 125, a plurality of cDNA strands are synthesized from the RNA templates obtained in step 120 using a strand-displacement reverse transcriptase. For example, a plurality of cDNA strands can be synthesized in an extension reaction using a plurality of random RNA primers (e.g., random hexamer primers, polyT primers, or a combination thereof). In general, any reverse transcriptase having strand-displacement activity can be used in the step. For example, in one embodiment, the reverse transcriptase enzyme is MMLV reverse transcriptase (available from Clontech). The reverse transcriptase with strong strand-displacement and template switching activity allows for multiple cDNA strand copies to be generated from a single RNA template, wherein each cDNA strand includes the unique sequence tag from the template switching oligonucleotide ligated to the 5'-end of the RNA template.

In step 130, optionally, a reverse complement DNA strand can be synthesized from the cDNA sequence to prepare a double-stranded DNA (dsDNA) sequencing library. A standard sequencing library preparation protocol (e.g., TRUSEQ^{®} library preparation protocol (Illumina, Inc.)), that includes the steps of end repair, 3'-end A-tailing, sequencing Y-adapter ligation, and PCR amplification, can be used to prepare the DNA sequencing library.

Aspects of the method further comprise sequencing at least a portion of a DNA sequencing library to obtain sequencing data or sequence reads (not shown). In general, any method known in the art can be used to obtain sequence data or sequence reads from the DNA sequencing library. For example, sequencing data or sequence reads can be acquired using next generation sequencing (NGS). Non-limiting examples of next-generation sequencing methods include: sequencing by synthesis technology (Illumina), pyrosequencing (454), ion semiconductor technology (Ion Torrent sequencing), single-molecule real-time sequencing (Pacific Biosciences), sequencing by ligation (SOLiD sequencing), and nanopore sequencing (Oxford Nanopore Technologies).

In another alternative embodiment, a fragmentation step may be used prior to preparation of a sequencing library (step 130 of method 100) to facilitate subsequent sequencing processes (e.g., cluster amplification prior to sequencing).

One or more steps of the method can be carried out in a single reaction step. For example, steps (b) through (d) may be carried out in a single reaction tube utilizing a reaction mixture comprising RNA primers (e.g., random hexamer RNA primers, polyT primers, or a combination thereof), a strand-displacement reverse transcriptase (e.g., MMLV reverse transcriptase), an RNA ligase (e.g., T4 RNA ligase), and optionally, a polynucleotide kinase (e.g., T4 polynucleotide kinase).

Also disclosed herein, but not encompassed within the scope of claims, but nevertheless considered useful for understanding the invention, is a method for preparing a sequencing library from a test sample comprising RNA, the method comprising the steps: (a) obtaining a test sample comprising one or more RNA sequences, and purifying the one or more RNA sequences from the test sample; (b) annealing a first RNA primer to the one or more RNA sequences; (c) extending the first RNA primer in a first nucleic acid extension reaction using reverse transcriptase, wherein the reverse transcriptase comprises reverse transcription and terminal transferase activities, to generate a plurality of DNA sequences complementary to the one or more RNA templates, and wherein the complementary DNA (cDNA) sequences further comprise a plurality of non-templated bases at the 3'-end of the cDNA sequences; (d) annealing a complementary nucleic acid sequence to the non-templated bases at the 3'-end of the cDNA sequence, wherein the complementary nucleic acid sequence further comprises a unique molecular identifier (UMI) or a unique sequence tag; (e) ligating the complementary nucleic acid sequence to the 5'-end of the RNA sequences to generate one or more RNA templates, wherein the one or more RNA templates comprise the original one or more RNA sequences covalently linked to the complementary nucleic acid sequence comprising the UMI or unique sequence tag; (f) annealing one or more second RNA primers to the RNA template; and (g) extending the one or more second RNA primers in a second nucleic acid extension reaction using a strand-displacement reverse transcriptase to generate a plurality of DNA sequence complementary to the RNA templates, wherein the plurality of complementary DNA (cDNA) each comprise the complementary DNA sequence and a UMI or unique sequence tag.

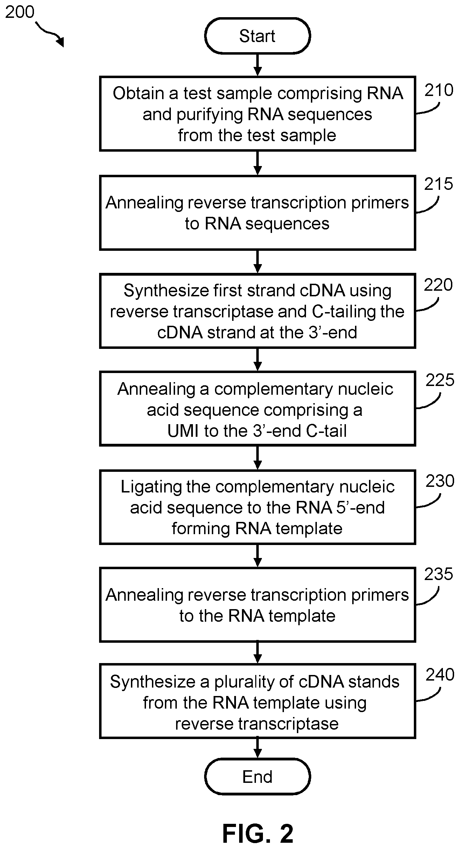

**FIG. 2** is a flow diagram illustrating a method for tagging and amplifying RNA sequences obtained from a test sample for preparation of a sequencing library. As shown, method 200 may include, but is not limited to, the following steps.

In step 210, an RNA-containing test sample is obtained and RNA sequences are purified from the test sample. The subject methods can be carried out on any suitable biological sample, as described in detail herein. A test sample may be a biological sample selected from the group consisting of blood, plasma, serum, urine, fecal, and saliva samples. In one preferred embodiment, a test sample is a blood sample. In one preferred method, a test sample is a plasma sample. In one method, the RNA sequences comprise cell-free RNA. Optionally, the 5'-ends of the RNA sequences are phosphorylated using T4 polynucleotide kinase. In another method, the RNA sequences may be fragmented after purification of the RNA sequences from the test sample.

In step 215, reverse transcription primers are annealed to the RNA sequences. In general, any known reverse transcription primers may be used. For example, the primers may be gene-specific primers or polyA primers. In another method, the reverse transcription primers are random hexamer primers. Although not wishing to be bound by theory, it is believed that the use of random hexamer primers in the reverse transcription reaction may allow all RNA molecules in the sample to be captured and used as template molecules for synthesis of first strand cDNA. Random priming also provides for greater coverage of a single RNA molecule.

In step 220, a first complementary DNA (cDNA) strand is synthesized and the 3'-end tailed with a non-templated base sequence using reverse transcriptase having both reverse transcription and terminal transferase activities. As described elsewhere in this application, the cDNA sequence is C-tailed. The reverse transcriptase has both strand-displacement and terminal transferase (e.g., C-tailing) activities. For example, in one embodiment, MMLV reverse transcriptase (available from Clontech) is used. In step 225, a complementary nucleic acid sequence to the non-templated bases is annealed (or hybridized to) the 3'-tail of the cDNA sequences, and subsequently ligated in step 230 to the 5'-ends of the RNA sequences to produce RNA templates using an RNA ligase. The complementary nucleic acid sequence (e.g., switch oligonucleotide) may include, for example, a complementary hybridization sequence (e.g., a poly-G tail), a unique sequence tag (e.g., a UMI sequence), and/or a universal primer sequence for initiating second strand cDNA synthesis. In general, any known RNA ligase can be used to ligate the template-switching oligonucleotide to the 5'-ends of the RNA sequence. In one method, the RNA ligase is T4 RNA ligase (available from New England BioLabs).

In step 235, one or more second RNA primers are annealed to the RNA template. The second RNA primers may comprise random hexamer primers, polyT primers, or a combination thereof.

In step 240, a plurality of cDNA strands are synthesized from the RNA templates obtained in step 120 using a strand-displacement reverse transcriptase. For example, a plurality of cDNA strands can be synthesized in an extension reaction using the RNA primers (e.g., random hexamer primers, polyT primers, or a combination thereof) annealed to the RNA templates in step 235. In general, any reverse transcriptase having strand-displacement activity can be used in the step. For example, the reverse transcriptase enzyme is MNLV reverse transcriptase (available from Clontech). In accordance with one method, the reverse transcriptase with strong strand-displacement and template switching activity allows for multiple cDNA strand copies to be generated from a single RNA template, wherein each cDNA strand includes the unique sequence tag from the template switching oligonucleotide ligated to the 5'-end of the RNA template.

Optionally, in a subsequent step (not shown), a reverse complement DNA strand may be synthesized from the cDNA sequence to prepare a double-stranded DNA (dsDNA) sequencing library. A standard sequencing library preparation protocol (e.g., TRUSEQ^{®} library preparation protocol (Illumina, Inc.)), that includes the steps of end repair, 3'-end A-tailing, sequencing Y-adapter ligation, and PCR amplification, can be used to prepare the DNA sequencing library. The method may further comprise (not show) sequencing at least a portion of DNA sequencing library to obtain sequencing data or sequence reads (not shown). In another alternative method, a fragmentation step may be used prior to preparation of a sequencing library (step 130 of method 100) to facilitate subsequent sequencing processes (e.g., cluster amplification prior to sequencing).

One or more steps of the method may be carried out in a single reaction step. For example, steps (b) through (g) may be carried out in a single reaction tube utilizing a reaction mixture comprising RNA primers (e.g., random hexamer RNA primers, polyT primers, or a combination thereof), a strand-displacement reverse transcriptase (e.g., MMLV reverse transcriptase), an RNA ligase (e.g., T4 RNA ligase), and optionally, a polynucleotide kinase (e.g., T4 polynucleotide kinase).

Also disclosed herein, but not explicitly claimed, are methods for preparing a sequencing library from a test sample comprising RNA molecules or sequences, the methods comprising the steps: (a) obtaining a test sample comprising one or more RNA sequences, and purifying the one or more RNA sequences from the test sample; (b) annealing a first RNA primer to the one or more RNA sequences; (c) extending the first RNA primer in a first nucleic acid extension reaction using a reverse transcriptase, wherein the reverse transcriptase comprises reverse transcription and terminal transferase activities, to generate a plurality of DNA sequences complementary to the one or more RNA templates, wherein the terminal transferase activity adds a cytosine (C) tail to the 3'-end of the complementary DNA (cDNA) sequences; (d) annealing a template switching oligonucleotide to the 3'-cytosine tail of the cDNA sequence, wherein the template switching oligonucleotide further comprises a unique molecular identifier (UMI) or a unique sequence tag; (e) ligating the template switching oligonucleotide to the 5'-end of the one or more RNA sequences with T4 RNA ligase to generate one or more RNA templates, wherein the one or more RNA templates comprise the original one or more RNA sequences covalently linked to the template switching oligonucleotide and the UMI or unique sequence tag; (f) annealing a plurality of second RNA primers to the one or more RNA templates; and (g) extending the plurality of second RNA primers in a second nucleic acid extension reaction using a strand-displacement reverse transcriptase to generate a plurality of DNA sequence complementary to the one or more RNA templates, wherein the plurality of complementary DNA (cDNA) each comprise the complementary DNA sequence and a UMI or unique sequence tag.

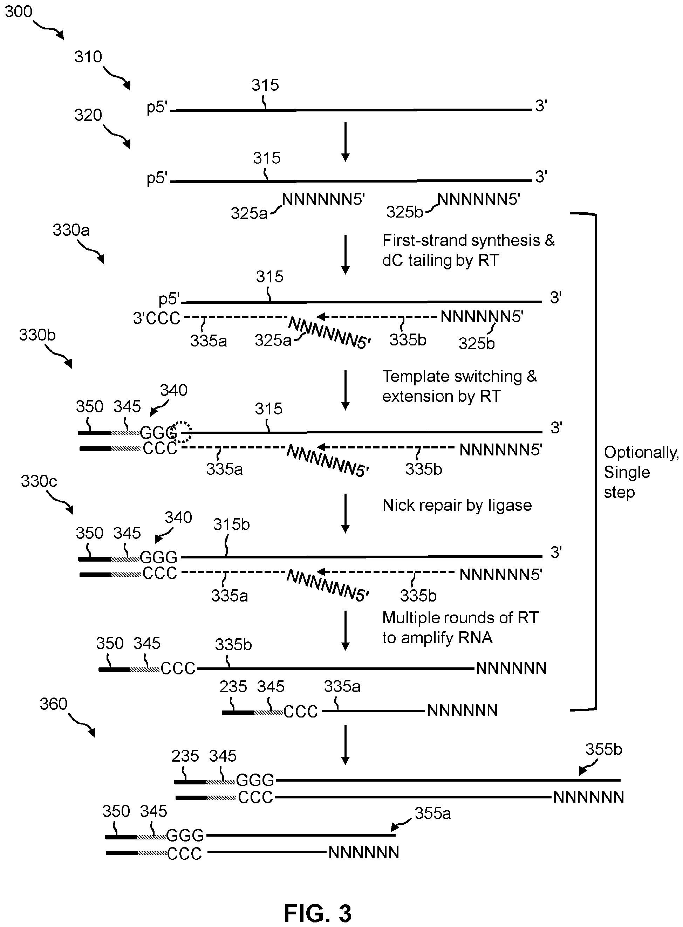

**FIG. 3** shows pictorially the steps of a method for tagging and amplifying RNA sequences obtained from a test sample for preparation of a sequencing library. Namely, in step 310, RNA-containing test sample is obtained and RNA sequences purified from the test sample. Optionally, the 5'-ends of the RNA sequences are phosphorylated using T4 polynucleotide kinase. The RNA sequences may be fragmented after purification of the RNA sequences from the test sample.

In step 320, one or more reverse transcriptase primers 325 are annealed to the RNA sequences 315. In one arrangement, the reverse transcriptase primers 325 are random hexamer primers. Any number of RT primers 325 can anneal along the length of the RNA sequence 315. In this example, 2 reverse transcriptase primers 325a and 325b are shown.

In step 330a, a population of first strand cDNA molecules 335 are synthesized from RNA sequences 315 in a reverse transcription reaction. Synthesis of first strand cDNA molecules 335 by the RT enzyme is initiated from RT primers 325 (e.g., as shown, random hexamer primers). In this example, two first strand cDNA molecules 335 (e.g., first strand cDNA molecules 335a and 335b) are shown. The strand displacement activity of the RT enzyme allows displacement of downstream first strand cDNA molecule 335a encountered during synthesis of first strand cDNA molecules 335b. When the RT enzyme reaches the 5'-end of RNA molecule 315, the RT enzyme adds a few non-templated deoxycytidines (e.g., as shown, CCC) to the 3'-end of cDNA molecule 335.

At step 330b, a template switching oligonucleotide 340 hybridizes to the cDNA strand 335 by base-pairing with the C tail (i.e., CCC) creating an extended RNA template that now includes UMI region 345, and optionally a universal primer region 350. As shown, the template switching oligonucleotide 340 may include, for example, an oligo (G) sequence (e.g., GGG), a UMI region 345, and a universal primer region 350. The nick (indicated by a dotted circle) between the template switching oligonucleotide 340 and RNA sequence 315 is repaired, or ligated, using an RNA ligase (e.g., T4 RNA ligase). Ligation of template switching oligonucleotide 340 to RNA sequence 315 covalently links the template switch oligonucleotide 340, and the UMI sequence 345, to the RNA molecule 315 creating an RNA template 315b for subsequent amplification of the original RNA sequence.

The RT enzyme extends first strand cDNA molecule 335 from the C-tail, creating a complementary UMI region 345, and the optional universal primer region 350 on first strand cDNA molecule 335. Because the template switching oligonucleotide 340 is covalently attached to the RNA template 315b, all cDNA strands 335 synthesized from the RNA template 315b will have the same UMI sequence (i.e., UMI region 345).

At step 330c, multiple rounds of reverse transcription and strand displacement can be performed to generate a plurality of cDNA sequences 335 from RNA template 315b, wherein each cDNA strand 335a and 335b, will have the same UMI sequence 345. As shown in Figure 3, the length of the cDNA strands 335a and 335b will vary depending on the location of the initiating RT primers 325.

Optionally, steps 330a, 330b, and 330c may be carried out in a single reaction, as shown in FIG. 3. The single reaction step may use a reaction mixture that includes RNA primers (e.g., random hexamer RNA primers, polyT primers, or a combination thereof), a reverse transcriptase (RT) enzyme with strand-displacement and terminal transferase activity, a template switching oligonucleotide having a UMI 345, and a ligase for ligation of the template switching oligonucleotide 340 to the 5'-ends of RNA sequence 315, creating RNA template 315b.

As shown in step 360, optionally, a reverse complement DNA strand may be synthesized from the cDNA sequence to prepare a double-stranded DNA (dsDNA) sequencing library. For example, a second DNA strand complementary to the cDNA may be synthesized in an extension reaction (e.g., using a DNA polymerase) from a DNA primer (not shown) that is complementary to universal primer region 350. A standard sequencing library preparation protocol (e.g., TRUSEQ^{®} library preparation protocol (Illumina, Inc.)), that includes the steps of end repair, 3'-end A-tailing, sequencing Y-adapter ligation, and PCR amplification, can be used to prepare the DNA sequencing library. The method further comprises sequencing at least a portion of a DNA sequencing library to obtain sequencing data or sequence reads (not shown).

### Sequencing and Bioinformatics

As reviewed above, aspects of the methods disclosed herein include sequencing of nucleic acid molecules to generate a plurality of sequence reads, compilation of a plurality of sequence reads into a sequencing library, and bioinformatic manipulation of the sequence reads and/or sequencing library to determine sequence information from a test sample (e.g., a biological sample). In some methods, one or more aspects of the subject methods are conducted using a suitably-programmed computer system, as described further herein.

In certain methods, a sample is collected from a subject, followed by enrichment for genetic regions or genetic fragments of interest. For example, a sample can be enriched by hybridization to a nucleotide array comprising cancer-related genes or gene fragments of interest. A sample can be enriched for genes of interest (e.g., cancer-associated genes) using other methods known in the art, such as hybrid capture. See, e.g., Lapidus (U.S. Patent Number 7,666,593). In one hybrid capture method, a solution-based hybridization method is used that includes the use of biotinylated oligonucleotides and streptavidin coated magnetic beads. See, e.g., Duncavage et al., J Mol Diagn. 13(3): 325-333 (2011); and Newman et al., Nat Med. 20(5): 548-554 (2014). Isolation of nucleic acid from a sample can be done according to any method known in the art.

Sequencing may be by any method or combination of methods known in the art. For example, known DNA sequencing techniques include, but are not limited to, classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, Polony sequencing, and SOLiD sequencing. Sequencing of separated molecules has more recently been demonstrated by sequential or single extension reactions using polymerases or ligases as well as by single or sequential differential hybridizations with libraries of probes.

One conventional method to perform sequencing is by chain termination and gel separation, as described by Sanger et al., Proc Natl. Acad. Sci. USA, 74(12): 5463 67 (1977). Another conventional sequencing method involves chemical degradation of nucleic acid fragments. See, Maxam et al., Proc. Natl. Acad. Sci., 74: 560 564 (1977). Methods have also been developed based upon sequencing by hybridization. See, e.g., Harris et al., (U.S. patent application number 2009/0156412).

A sequencing technique that can be used in the methods disclosed herein includes, for example, Helicos True Single Molecule Sequencing (tSMS) (Harris T. D. et al. (2008) Science 320:106-109). Further description of tSMS is shown, for example, in Lapidus et al. (U.S. patent number 7,169,560), Lapidus et al. (U.S. patent application publication number 2009/0191565), Quake et al. (U.S. patent number 6,818,395), Harris (U.S. patent number 7,282,337), Quake et al. (U.S. patent application publication number 2002/0164629), and Braslavsky, et al., PNAS (USA), 100: 3960-3964 (2003).

Another example of a DNA sequencing technique that can be used in the methods disclosed herein is 454 sequencing (Roche) (Margulies, M et al. 2005, Nature, 437, 376-380). Another example of a DNA sequencing technique that can be used in the methods disclosed herein is SOLiD technology (Applied Biosystems). Another example of a DNA sequencing technique that can be used in the methods disclosed herein is Ion Torrent sequencing (U.S. patent application publication numbers 2009/0026082, 2009/0127589, 2010/0035252, 2010/0137143, 2010/0188073, 2010/0197507, 2010/0282617, 2010/0300559, 2010/0300895, 2010/0301398, and 2010/0304982).

In some methods, the sequencing technology is Illumina sequencing. Illumina sequencing is based on the amplification of DNA on a solid surface using fold-back PCR and anchored primers. Genomic DNA can be fragmented, or in the case of cfDNA, fragmentation is not needed due to the already short fragments. Adapters are ligated to the 5'- and 3'-ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a sequencing technology that can be used in the methods disclosed herein includes the single molecule, real-time (SMRT) technology of Pacific Biosciences. Yet another example of a sequencing technique that can be used in the methods disclosed herein is nanopore sequencing (Soni G V and Meller A. (2007) Clin Chem 53: 1996-2001). Another example of a sequencing technique that can be used in the methods disclosed herein involves using a chemical-sensitive field effect transistor (chemFET) array to sequence DNA (for example, as described in US Patent Application Publication No. 20090026082). Another example of a sequencing technique that can be used in the methods disclosed herein involves using an electron microscope (Moudrianakis E. N. and Beer M. Proc Natl Acad Sci USA. 1965 March; 53:564-71).

If the nucleic acid from the sample is degraded or only a minimal amount of nucleic acid can be obtained from the sample, PCR can be performed on the nucleic acid in order to obtain a sufficient amount of nucleic acid for sequencing (See, e.g., Mullis et al. U.S. patent number 4,683,195).

### Biological Samples

Aspects of the methods disclosed herein involve obtaining a test sample, e.g., a biological sample, such as a tissue and/or body fluid sample, from a subject for purposes of analyzing a plurality of nucleic acids (e.g., a plurality of RNA molecules) therein. Samples can be collected in any clinically-acceptable manner. Any test sample suspected of containing a plurality of nucleic acids can be used in conjunction with the methods disclosed herein. In some methods, a test sample can comprise a tissue, a body fluid, or a combination thereof. In some methods, a biological sample is collected from a healthy subject. In some methods, a biological sample is collected from a subject who is known to have a particular disease or disorder (e.g., a particular cancer or tumor). In some methods, a biological sample is collected from a subject who is suspected of having a particular disease or disorder.

As used herein, the term "tissue" refers to a mass of connected cells and/or extracellular matrix material(s). Non-limiting examples of tissues that are commonly used in conjunction with the present methods include skin, hair, finger nails, endometrial tissue, nasal passage tissue, central nervous system (CNS) tissue, neural tissue, eye tissue, liver tissue, kidney tissue, placental tissue, mammary gland tissue, gastrointestinal tissue, musculoskeletal tissue, genitourinary tissue, bone marrow, and the like, derived from, for example, a human or non-human mammal. Tissue samples can be prepared and provided in the form of any tissue sample types known in the art, such as, for example and without limitation, formalin-fixed paraffin-embedded (FFPE), fresh, and fresh frozen (FF) tissue samples.

As used herein, the term "body fluid" refers to a liquid material derived from a subject, e.g., a human or non-human mammal. Non-limiting examples of body fluids that are commonly used in conjunction with the present methods include mucous, blood, plasma, serum, serum derivatives, synovial fluid, lymphatic fluid, bile, phlegm, saliva, sweat, tears, sputum, amniotic fluid, menstrual fluid, vaginal fluid, semen, urine, cerebrospinal fluid (CSF), such as lumbar or ventricular CSF, gastric fluid, a liquid sample comprising one or more material(s) derived from a nasal, throat, or buccal swab, a liquid sample comprising one or more materials derived from a lavage procedure, such as a peritoneal, gastric, thoracic, or ductal lavage procedure, and the like.

In some methods, a test sample can comprise a fine needle aspirate or biopsied tissue. In some methods, a test sample can comprise media containing cells or biological material. In some methods, a test sample can comprise a blood clot, for example, a blood clot that has been obtained from whole blood after the serum has been removed. In some methods, a test sample can comprise stool. In one preferred method, a test sample is drawn whole blood. In one aspect, only a portion of a whole blood sample is used, such as plasma, red blood cells, white blood cells, and platelets. In some methods, a test sample is separated into two or more component parts in conjunction with the present methods. For example, in some methods, a whole blood sample is separated into plasma, red blood cell, white blood cell, and platelet components.

In some methods, a test sample includes a plurality of nucleic acids not only from the subject from which the test sample was taken, but also from one or more other organisms, such as viral DNA/RNA that is present within the subject at the time of sampling.

Nucleic acid can be extracted from a test sample according to any suitable methods known in the art, and the extracted nucleic acid can be utilized in conjunction with the methods described herein. See, e.g., Maniatis, et al., Molecular Cloning: A Laboratory Manual, Cold Spring Harbor, N.Y., pp. 280-281, 1982.

In one preferred method, cell free nucleic acid (e.g., cell-free DNA (cfDNA) and/or cell-free RNA (cfRNA)) are extracted from a test sample. cfDNA are short base nuclear-derived DNA fragments present in several bodily fluids (e.g. plasma, stool, urine). See, e.g., Mouliere and Rosenfeld, PNAS 112(11): 3178-3179 (Mar 2015); Jiang et al., PNAS (Mar 2015); and Mouliere et al., Mol Oncol, 8(5):927-41 (2014). Tumor-derived circulating tumor nucleic acids (e.g., ctDNA and/or ctRNA) constitutes a minority population of cfNAs (i.e., cfDNA and/or cfRNA), in some cases, varying up to about 50%. In some methods, ctDNA and/or ctRNA varies depending on tumor stage and tumor type. In some methods, ctDNA and/or ctRNA varies from about 0.001% up to about 30%, such as about 0.01% up to about 20%, such as about 0.01% up to about 10%. The covariates of ctDNA and/or ctRNA are not fully understood, but appear to be positively correlated with tumor type, tumor size, and tumor stage. E.g., Bettegowda et al, Sci Trans Med, 2014; Newmann et al, Nat Med, 2014. Despite the challenges associated with the low population of ctDNA/ctRNA in cfNAs, tumor variants have been identified in ctDNA and/or ctRNA across a wide span of cancers. E.g., Bettegowda et al, Sci Trans Med, 2014. Furthermore, analysis of cfDNA and/or cfRNA versus tumor biopsy is less invasive, and methods for analyzing, such as sequencing, enable the identification of sub-clonal heterogeneity. Analysis of cfDNA and/or cfRNA has also been shown to provide for more uniform genome-wide sequencing coverage as compared to tumor tissue biopsies. In some methods, a plurality of cfDNA and/or cfRNA are extracted from a sample in a manner that reduces or eliminates co-mingling of cfDNA and genomic DNA. For example, in some methods, a sample is processed to isolate a plurality of the cfDNA and/or cfRNA therein in less than about 2 hours, such as less than about 1.5, 1 or 0.5 hours.

A non-limiting example of a procedure for preparing nucleic acid from a blood sample follows. Blood may be collected in 10mL EDTA tubes (for example, the BD VACUTAINER^{®} family of products from Becton Dickinson, Franklin Lakes, New Jersey), or in collection tubes that are adapted for isolation of cfDNA (for example, the CELL FREE DNA BCT^{®} family of products from Streck, Inc., Omaha, Nebraska) can be used to minimize contamination through chemical fixation of nucleated cells, but little contamination from genomic DNA is observed when samples are processed within 2 hours or less, as is the case in some of the present methods. Beginning with a blood sample, plasma may be extracted by centrifugation, e.g., at 3000rpm for 10 minutes at room temperature minus brake. Plasma may then be transferred to 1.5ml tubes in 1ml aliquots and centrifuged again at 7000rpm for 10 minutes at room temperature. Supernatants can then be transferred to new 1. 5ml tubes. At this stage, samples can be stored at -80°C. In certain embodiments, samples can be stored at the plasma stage for later processing, as plasma may be more stable than storing extracted cfDNA and/or cfRNA.

Plasma DNA and/or RNA can be extracted using any suitable technique. For example, plasma DNA and/or RNA can be extracted using one or more commercially available assays, for example, the QIAmp Circulating Nucleic Acid Kit family of products (Qiagen N.V., Venlo Netherlands). In certain methods, the following modified elution strategy may be used. DNA and/or RNA may be extracted using, e.g., a QIAmp Circulating Nucleic Acid Kit, following the manufacturer's instructions (maximum amount of plasma allowed per column is 5mL). If cfDNA and/or cfRNA are being extracted from plasma where the blood was collected in Streck tubes, the reaction time with proteinase K may be doubled from 30 min to 60 min. Preferably, as large a volume as possible should be used (i.e., 5mL). In various methods, a two-step elution may be used to maximize cfDNA and/or cfRNA yield. First, DNA and/or RNA can be eluted using 30µL of buffer AVE for each column. A minimal amount of buffer necessary to completely cover the membrane can be used in the elution in order to increase cfDNA and/or cfRNA concentration. By decreasing dilution with a small amount of buffer, downstream desiccation of samples can be avoided to prevent melting of double stranded DNA or material loss. Subsequently, about 30µL of buffer for each column can be eluted. In some embodiments, a second elution may be used to increase DNA and/or RNA yield.

In other methods, RNA can be extracted and/or isolated using any suitable technique. For example, RNA can be extracted using a commercially-available kit and/or protocol, e.g., a QIAamp Circulating Nucleic Acids kit and micro RNA extraction protocol.

In some methods, the methods involve DNase treating an extracted nucleic acid sample to remove cell-free DNA from a mixed cfDNA and cfRNA test sample.

### Computer Systems and Devices (not claimed)

Aspects of the methods described herein can be performed using any type of computing device, such as a computer, that includes a processor, e.g., a central processing unit, or any combination of computing devices where each device performs at least part of the process or method. Systems and methods described herein may be performed with a handheld device, e.g., a smart tablet, or a smart phone, or a specialty device produced for the system.

Methods disclosed herein can be performed using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections).

Processors suitable for the execution of computer programs include, by way of example, both general and special purpose microprocessors, and any one or more processors of any kind of digital computer. Generally, a processor will receive instructions and data from a read-only memory or a random access memory, or both. The essential elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including, by way of example, semiconductor memory devices, (e.g., EPROM, EEPROM, solid state drive (SSD), and flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can be implemented on a computer having an I/O device, e.g., a CRT, LCD, LED, or projection device for displaying information to the user and an input or output device such as a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The subject matter described herein can be implemented in a computing system that includes a back-end component (e.g., a data server), a middleware component (e.g., an application server), or a front-end component (e.g., a client computer having a graphical user interface or a web browser through which a user can interact with an implementation of the subject matter described herein), or any combination of such back-end, middleware, and front-end components. The components of the system can be interconnected through a network by any form or medium of digital data communication, e.g., a communication network. For example, a reference set of data may be stored at a remote location and a computer can communicate across a network to access the reference data set for comparison purposes. In other arrangements, however, a reference data set can be stored locally within the computer, and the computer accesses the reference data set within the CPU for comparison purposes. Examples of communication networks include, but are not limited to, cell networks (e.g., 3G or 4G), a local area network (LAN), and a wide area network (WAN), e.g., the Internet.

The subject matter described herein can be implemented as one or more computer program products, such as one or more computer programs tangibly embodied in an information carrier (e.g., in a non-transitory computer-readable medium) for execution by, or to control the operation of, a data processing apparatus (e.g., a programmable processor, a computer, or multiple computers). A computer program (also known as a program, software, software application, app, macro, or code) can be written in any form of programming language, including compiled or interpreted languages (e.g., C, C++, Perl), and it can be deployed in any form, including as a stand-alone program or as a module, component, subroutine, or other unit suitable for use in a computing environment. Systems and methods of the invention can include instructions written in any suitable programming language known in the art, including, without limitation, C, C++, Perl, Java^{®}, ActiveX^{®}, HTML5, Visual Basic^{®}, or JavaScript^{®}.

A computer program does not necessarily correspond to a file. A program can be stored in a file or a portion of a file that holds other programs or data, in a single file dedicated to the program in question, or in multiple coordinated files (e.g., files that store one or more modules, subprograms, or portions of code). A computer program can be deployed to be executed on one computer or on multiple computers at one site or distributed across multiple sites and interconnected by a communication network.

A file can be a digital file, for example, stored on a hard drive, SSD, CD, or other tangible, non-transitory medium. A file can be sent from one device to another over a network (e.g., as packets being sent from a server to a client, for example, through a Network Interface Card, modem, wireless card, or similar).

Writing a file involves transforming a tangible, non-transitory computer-readable medium, for example, by adding, removing, or rearranging particles (e.g., with a net charge or dipole moment into patterns of magnetization by read/write heads), the patterns then representing new collocations of information about objective physical phenomena desired by, and useful to, the user. In some arrangements, writing involves a physical transformation of material in tangible, non-transitory computer readable media (e.g., with certain optical properties so that optical read/write devices can then read the new and useful collocation of information, e.g., burning a CD-ROM). In some arrangements, writing a file includes transforming a physical flash memory apparatus such as NAND flash memory device and storing information by transforming physical elements in an array of memory cells made from floating-gate transistors. Methods of writing a file are well-known in the art and, for example, can be invoked manually or automatically by a program or by a save command from software or a write command from a programming language.

Suitable computing devices typically include mass memory, at least one graphical user interface, at least one display device, and typically include communication between devices. The mass memory illustrates a type of computer-readable media, namely computer storage media. Computer storage media may include volatile, nonvolatile, removable, and non-removable media implemented in any method or technology for storage of information, such as computer readable instructions, data structures, program modules, or other data. Examples of computer storage media include RAM, ROM, EEPROM, flash memory, or other memory technology, CD-ROM, digital versatile disks (DVD) or other optical storage, magnetic cassettes, magnetic tape, magnetic disk storage or other magnetic storage devices, Radiofrequency Identification (RFID) tags or chips, or any other medium that can be used to store the desired information, and which can be accessed by a computing device.

Functions described herein can be implemented using software, hardware, firmware, hardwiring, or combinations of any of these. Any of the software can be physically located at various positions, including being distributed such that portions of the functions are implemented at different physical locations.

As one skilled in the art would recognize as necessary or best-suited for performance of the methods disclosed herein, a computer system for implementing some or all of the described methods can include one or more processors (e.g., a central processing unit (CPU) a graphics processing unit (GPU), or both), main memory and static memory, which communicate with each other via a bus.

A processor will generally include a chip, such as a single core or multi-core chip, to provide a central processing unit (CPU). A process may be provided by a chip from Intel^{®} or AMD^{®}.

Memory can include one or more machine-readable devices on which is stored one or more sets of instructions (e.g., software) which, when executed by the processor(s) of any one of the disclosed computers can accomplish some or all of the methodologies or functions described herein. The software may also reside, completely or at least partially, within the main memory and/or within the processor during execution thereof by the computer system. Preferably, each computer includes a non-transitory memory such as a solid state drive, flash drive, disk drive, hard drive, etc.

While the machine-readable devices can be a single medium, the term "machine-readable device" should be taken to include a single medium or multiple media (e.g., a centralized or distributed database, and/or associated caches and servers) that store the one or more sets of instructions and/or data. These terms shall also be taken to include any medium or media that are capable of storing, encoding, or holding a set of instructions for execution by the machine and that cause the machine to perform any one or more of the methodologies of the present invention. These terms shall accordingly be taken to include, but not be limited to, one or more solid-state memories (e.g., subscriber identity module (SIM) card, secure digital card (SD card), micro SD card, or solid-state drive (SSD)), optical and magnetic media, and/or any other tangible storage medium or media.

A computer will generally include one or more I/O device such as, for example, one or more of a video display unit (e.g., a liquid crystal display (LCD) or a cathode ray tube (CRT)), an alphanumeric input device (e.g., a keyboard), a cursor control device (e.g., a mouse), a disk drive unit, a signal generation device (e.g., a speaker), a touchscreen, an accelerometer, a microphone, a cellular radio frequency antenna, and a network interface device, which can be, for example, a network interface card (NIC), Wi-Fi card, or cellular modem.

Any of the software can be physically located at various positions, including being distributed such that portions of the functions are implemented at different physical locations.

Additionally, systems can be provided to include reference data. Any suitable genomic data may be stored for use within the system. Examples include, but are not limited to: comprehensive, multi-dimensional maps of the key genomic changes in major types and subtypes of cancer from The Cancer Genome Atlas (TCGA); a catalog of genomic abnormalities from The International Cancer Genome Consortium (ICGC); a catalog of somatic mutations in cancer from COSMIC; the latest builds of the human genome and other popular model organisms; up-to-date reference SNPs from dbSNP; gold standard indels from the 1000 Genomes Project and the Broad Institute; exome capture kit annotations from Illumina, Agilent, Nimblegen, and Ion Torrent; transcript annotations; small test data for experimenting with pipelines (e.g., for new users).

In some arrangements, data is made available within the context of a database included in a system. Any suitable database structure may be used including relational databases, object-oriented databases, and others. In some arrangements, reference data is stored in a relational database such as a "not-only SQL" (NoSQL) database. In certain arrangements, a graph database is included within systems of the invention. It is also to be understood that the term "database" as used herein is not limited to one single database; rather, multiple databases can be included in a system. For example, a database can include two, three, four, five, six, seven, eight, nine, ten, fifteen, twenty, or more individual databases, including any integer of databases therein. For example, one database can contain public reference data, a second database can contain test data from a patient, a third database can contain data from healthy individuals, and a fourth database can contain data from sick individuals with a known condition or disorder. It is to be understood that any other configuration of databases with respect to the data contained therein is also contemplated by the methods described herein.

The subject matter herein contains important information, exemplification and guidance that can be adapted to the practice of this invention in its various embodiments and equivalents thereof.

This specification is divided into sections for the convenience of the reader only. Headings should not be construed as limiting of the scope of the invention. The definitions are intended as a part of the description of the invention.

## Claims

1. A method for preparing a sequencing library from a test sample comprising RNA, the method comprising the steps:
(a) purifying RNA sequences from a test sample;
(b) synthesizing first complementary DNA (cDNA) strands based on the RNA sequences and C-tailing 3 '-end of cDNA strand;
(c) annealing a complementary template switching oligonucleotide to the C-tail of the cDNA and ligating the complementary template switching oligonucleotide to the 5'-ends of the RNA sequences to produce RNA templates; and
(d) synthesizing a plurality of cDNA strands from the RNA templates using a strand-displacement reverse transcriptase.

2. The method of claim 1, wherein the test sample comprises cell-free RNA (cfRNA).

3. The method of claim 2, wherein the cfRNA-comprising test sample is from a whole blood, a blood fraction, plasma, serum, urine, fecal, saliva, a tissue biopsy, pleural fluid, pericardial fluid, cerebral spinal fluid, or peritoneal fluid test sample.

4. The method of any one of the preceding claims, wherein the one or more RNA sequences are fragmented after purification of the one or more RNA sequences from the test sample.

5. The method of claim 1, wherein the first cDNA strand synthesis (step (b)) utilizes a first primer, and wherein the second cDNA strand synthesis (step (d)) utilizes a second primer.

6. The method of claim 5, wherein the first and/or second RNA primers are random hexamer primers.

7. The method of claim 5 wherein the first and/or second RNA primers are poly T primers.

8. The method of any one of the preceding claims, wherein the reverse transcriptase is MMLV reverse transcriptase.

9. The method of any one of the preceding claims, wherein the ligation step utilizes T4 RNA ligase.

10. The method of claim 1, wherein reverse complement DNA strands are synthesized from the plurality of cDNA strands obtained in step (d) to generate a dsDNA sequencing library.

11. The method of claim10, wherein the DNA library is sequenced.

12. The method of claim 11, wherein a UMI or unique sequence tag are used for error correction.

## Patentansprüche

1. Ein Verfahren zur Herstellung einer Sequenzierungsbibliothek aus einer Testprobe, die RNA beinhaltet, wobei das Verfahren die folgenden Schritte beinhaltet:
(a) Aufreinigen von RNA-Sequenzen aus einer Testprobe;
(b) Synthetisieren erster komplementärer DNA(cDNA)-Stränge basierend auf den RNA-Sequenzen und Versehen des 3'-Endes des cDNA-Strangs mit einem C-Schwanz;
(c) Anlagern eines komplementären Matrizenwechsel-Oligonukleotids an den C-Schwanz der cDNA and Ligieren des komplementären Matrizenwechsel-Oligonukleotids an die 5'-Enden der RNA-Sequenzen, um RNA-Matrizen zu produzieren; und
(d) Synthetisieren einer Vielzahl von cDNA-Strängen aus den RNA-Matrizen unter Verwendung einer Strangverdrängungs-Reverse-Transkriptase.

2. Verfahren gemäß Anspruch 1, wobei die Testprobe zellfreie RNA (cfRNA) beinhaltet.

3. Verfahren gemäß Anspruch 2, wobei die cfRNA beinhaltende Testprobe aus einer Vollblut-, einer Blutfraktions-, Plasma-, Serum-, Urin-, Fäkal-, Speichel-, einer Gewebebiopsie-, Pleuraflüssigkeits-, Perikardflüssigkeits-, Liquor-cerebrospinalis- oder Peritonealflüssigkeitstestprobe stammt.

4. Verfahren gemäß einem der vorhergehenden Ansprüche, wobei die eine oder mehreren RNA-Sequenzen nach Aufreinigung der einen oder mehreren RNA-Sequenzen aus der Testprobe fragmentiert werden.

5. Verfahren gemäß Anspruch 1, wobei die erste cDNA-Strangsynthese (Schritt (b)) einen ersten Primer einsetzt und wobei die zweite cDNA-Strangsynthese (Schritt (d)) einen zweiten Primer einsetzt.

6. Verfahren gemäß Anspruch 5, wobei der erste und/oder zweite RNA-Primer Zufallshexamerprimer sind.

7. Verfahren gemäß Anspruch 5, wobei der erste und/oder zweite RNA-Primer Poly-T-Primer sind.

8. Verfahren gemäß einem der vorhergehenden Ansprüche, wobei die reverse Transkriptase MMLV-Reverse-Transkriptase ist.

9. Verfahren gemäß einem der vorhergehenden Ansprüche, wobei der Ligationsschritt T4-RNA-Ligase einsetzt.

10. Verfahren gemäß Anspruch 1, wobei Reverse-Komplement-DNA-Stränge aus der Vielzahl von in Schritt (d) erhaltenen cDNA-Stränge synthetisiert werden, um eine dsDNA-Sequenzierungsbibliothek zu erzeugen.

11. Verfahren gemäß Anspruch 10, wobei die DNA-Bibliothek sequenziert wird.

12. Verfahren gemäß Anspruch 11, wobei ein UMI oder eindeutiges Sequenztag zur Fehlerkorrektur verwendet wird.

## Revendications

1. Un procédé pour la préparation d'une bibliothèque de séquençage à partir d'un échantillon d'essai comprenant de l'ARN, le procédé comprenant les étapes :
(a) de purification de séquences d'ARN à partir d'un échantillon d'essai ;
(b) de synthèse de premiers brins d'ADN complémentaire (ADNc) sur la base des séquences d'ARN et d'ajout d'une queue C à l'extrémité 3' du brin d'ADNc ;
(c) d'hybridation d'un oligonucléotide de commutation de matrice (TSO, *Template Switching Oligonucleotide*) complémentaire à la queue C de l'ADNc et de ligature de l'oligonucléotide de commutation de matrice complémentaire aux extrémités 5' des séquences d'ARN afin de produire des matrices d'ARN ; et
(d) de synthèse d'une pluralité de brins d'ADNc à partir des matrices d'ARN à l'aide d'une transcriptase inverse par déplacement de brin.

2. Le procédé de la revendication 1, dans lequel l'échantillon d'essai comprend de l'ARN acellulaire (ARNcf).

3. Le procédé de la revendication 2, dans lequel l'échantillon d'essai comprenant de l'ARNcf provient d'un échantillon d'essai de sang total, de fraction sanguine, de plasma, de sérum, d'urine, de matière fécale, de salive, de biopsie tissulaire, de fluide pleural, de fluide péricardique, de fluide cérébrospinal, ou de fluide péritonéal.

4. Le procédé de l'une quelconque des revendications précédentes, dans lequel les une ou plusieurs séquences d'ARN sont fragmentées après purification des une ou plusieurs séquences d'ARN à partir de l'échantillon d'essai.

5. Le procédé de la revendication 1, dans lequel la synthèse de premiers brins d'ADNc (étape (b)) emploie une première amorce, et dans lequel la synthèse de deuxièmes brins d'ADNc (étape (d)) emploie une deuxième amorce.

6. Le procédé de la revendication 5, dans lequel les première et/ou deuxième amorces d'ARN sont des amorces hexamères aléatoires.

7. Le procédé de la revendication 5 dans lequel les première et/ou deuxième amorces d'ARN sont des amorces poly T.

8. Le procédé de l'une quelconque des revendications précédentes, dans lequel la transcriptase inverse est la transcriptase inverse MMLV.

9. Le procédé de l'une quelconque des revendications précédentes, dans lequel l'étape de ligature emploie de l'ARN ligase T4.

10. Le procédé de la revendication 1, dans lequel des brins d'ADN complémentaires inverses sont synthétisés à partir de la pluralité de brins d'ADNc obtenus à l'étape (d) afin d'engendrer une bibliothèque de séquençage d'ADNds.

11. Le procédé de la revendication 10, dans lequel la bibliothèque d'ADN est séquencée.

12. Le procédé de la revendication 11, dans lequel un UMI ou marqueur de séquence unique est utilisé pour une correction d'erreurs.
